# EUROPEAN PATENT SPECIFICATION

(11) **EP 0 789 598 B1**
(45) Date of publication and mention of the grant of the patent: **06.05.1998**
(21) Application number: 94931111.2
(22) Date of filing: 28.10.1994
(51) Int. Cl.: A61M 25/00, A61M 25/10

(54) **CATHETERS AND THEIR MANUFACTURE**
KATHETER UND HERSTELLUNGSVERFAHREN
CATHETERS ET LEUR FABRICATION

(30) Priority: 10.11.1993 GB 9323143
(43) Date of publication of application: 20.08.1997
(73) Proprietor: Smiths Industries Public Limited Company, London, NW11 8DS (GB)
(72) Inventor: COLLINS, Michael, Norman, Folkestone Kent CT18 8HD (GB)
(74) Representative: Flint, Jonathan McNeill
(86) International application number: GB9402367
(87) International publication number: WO9513109

(56) References cited:
- EP-A- 0 371 497
- EP-A- 0 485 903
- DE-A- 2 916 097
- US-A- 3 460 540

## Description

This invention relates to catheters of the kind having a shaft with a main lumen, a minor lumen extending along a rib projecting internally into the main lumen and a coupling into which the shaft is bonded at its proximal end, the coupling making connection with the minor lumen via an opening in the wall of the shaft.

The invention also relates to methods of manufacture of catheters.

Many catheters have a shaft extruded with a small-bore minor lumen extending along its length within the thickness of the wall. Where the minor lumen needs to have a relatively large cross section, the minor lumen can extend within an integral rib projecting internally of the catheter. The lumen may, for example, be used to supply inflation fluid to an inflatable cuff or for other purposes, such as irrigation. At the proximal end of the catheter there is some means for making access to the minor lumen, such as a coupling into which the main body of the catheter is bonded. Access to the minor lumen is made by an opening cut through the outside wall of the catheter into the lumen, the lumen being closed proximally of the opening by means of a plug inserted into the lumen, such as described in US-A-3460540. This can present a problem because insertion of the shaft into the coupling tends to deform the proximal end of the shaft. The additional wall thickness across the plugged rib makes this part of the wall stiffer and causes the rib to be squeezed inwardly of the shaft. This reduces the cross section of the main lumen through the catheter at the proximal end and reduces flow rates through the catheter. The problem is particularly severe in catheters with two minor lumens since these are generally located diametrically opposite one another and will be deformed together by the action of bonding into the coupling.

It is an object of the present invention to provide a catheter and method of manufacture by which this problem can be alleviated.

According to one aspect of the present invention there is provided a catheter of the above-specified kind, characterised in that the rib at the machine end is heat formed outwardly into the wall of the shaft so as to occlude the minor lumen.

The catheter may have an inflatable cuff encircling the shaft close to the patient end, the minor lumen opening into the cuff. The shaft preferably has two minor lumens extending along respective ribs projecting internally into the main lumen, both ribs being formed outwardly at the machine end into the wall of the shaft so as to occlude both lumens, and the coupling making connection with both minor lumens via respective openings in the wall of the shaft. The coupling may have a main body making connection with the main lumen and two side arms making connection with respective ones of the minor lumens. The patient end tip of the catheter may be closed, the catheter having an eye cutting through the or a minor lumen so that both the main lumen and the minor lumen open through the eye. The two minor lumens are preferably diametrically opposite one another, the catheter having two eyes diametrically opposite one another and cutting through both minor lumens, one of the minor lumens being closed on the machine side of its eye. The catheter may have two further eyes displaced by 90° relative to the first two eyes so that the further two eyes do not cut the minor lumens.

According to another aspect of the present invention there is provided a method of manufacture of a catheter including the steps of providing a shaft with a main lumen and a minor lumen extending along a rib projecting internally into the main lumen, occluding the minor lumen, forming art opening into the minor lumen through the wall of the catheter at a point distally of the occlusion, and bonding the machine end of the shaft into a coupling, characterised in that the minor lumen is occluded by heat forming the interior of the shaft at the machine end to mould the rib outwardly into the wall of the shaft.

The shaft may be heat formed by pushing the machine end of the shaft into a heated die having a central core.

A urinary catheter and its method of manufacture will now be described, by way of example, with reference to the accompanying drawings, in which:
- Figure 1: is a partly sectional side elevation view of the catheter;
- Figure 2: is a lateral section across the catheter along the line II - II;
- Figure 3: is a lateral section across the catheter along the line III - III;
- Figure 4: is a cross section showing a step in the manufacture of the catheter; and
- Figure 5: is a sectional view of a part of the catheter after the step shown in Figure 4.

The catheter is a urological catheter and is about 400 mm long with an external diameter of about 7 mm.

The catheter comprises a main tubular shaft 1 bonded at its proximal or machine end 2 into a coupling 3. The tubular shaft 1 is extruded from a flexible plastics material and has a main bore or lumen 10 and two minor lumens 11 and 12. The minor lumens extend along the wall of the body within respective internally-projecting ribs 13 and 14 located diametrically opposite one another end and extruded integrally with the shaft.

At its distal, patient end 15, the shaft 1 is rounded and closed but is provided with a pair of eyes or openings 16 and 17 in the wall of the shaft located diametrically opposite one another close to the end. One of the eyes 16 cuts through a first of the minor lumens 11 so that the lumen opens into the eye. The other eye 17 cuts through the second minor lumen 12 but this is sealed closed by a plug 18 on the proximal, machine side of the eye.

A second pair of eyes 19 and 20 is located just proximally of the eyes 16 and 17. The eyes 19 and 20 are located diametrically opposite one another but are displaced around the shalt 1 by 90° relative to the eyes 16 and 17 so that they lie between the two minor lumens 11 and 12 and do not cut through them.

An inflatable latex cuff 21 encircles the shaft 1 just to the rear of the eyes 19 and 20, and is bonded to the shaft at its opposite ends 22 and 23. The second minor lumen 12 opens into the cuff 21 through an opening 24 cut into the wall of the shaft from the outer surface, overlying the lumen.

At the machine end 2 of the shaft 1, both minor lumens 11 and 12 are sealed closed by heat forming the ribs 13 and 14 outwardly (in a manner described later). This gives the interior of the shaft a circular shape at the end and a gradual transition over a distance of about 10mm to the full projecting profile of the ribs. Two openings 25 and 26 are cut into respective ones of the minor lumens 11 and 12 from the outside of the shaft 1 at a point about 14mm from the proximal end, that is, distally of the occlusion.

The coupling 3 is a plastics moulding of trident shape with a main, central tubular body 30, about 70 mm long and with two side arms 31 and 32 joined to the central body close to its patient end. The central body 30 has a circular passage 33 extending along its length. A distal portion 34 of the passage extends for about 10mm and has the same diameter as the exterior of the shaft 1. The passage 33 continues as an intermediate portion 35, about 17 mm long, which tapers to a reduced diameter less than that of the shaft. The proximal portion 36 of the passage 33 tapers to an increased diameter of about 10mm over a distance of about 40 cm. Reinforcing ribs 37 extend around the outside of the central body 30.

The machine end 2 of the shaft 1 is inserted into the central body 30 so that it is located in the tapering, intermediate portion 35 of the passage 33. Because the diameter of the intermediate portion 35 is less than the external diameter of the shaft 1, the shaft is deformed inwardly and the main lumen through the shaft is constricted slightly. The shaft 1 is bonded into the coupling 3 by means of a solvent or by heat. The central body 30 is connected, in use, to a suitable coupling (not shown) at the end of urine drainage tubing extending to a drainage receptacle.

The side arm 31 opens into the central body 30, in the distal portion 34, in alignment with the opening 26 into the cuff-inflation lumen 12. The side arm 31 extends proximally alongside the central body 30 and is provided with a one-way valve 38 and a female coupling 39 into which the nose of an inflation syringe can be inserted.

The other side arm 32 opens into the central body 30 diametrically opposite the inflation arm 31, in alignment with the opening 25 into the minor lumen 11. The side arm 32 is used for supplying irrigating liquid to the catheter and has a female coupling 40 at its proximal end that can be connected to a suitable supply of liquid.

In use, the catheter is inserted in the bladder and the cuff 21 is inflated with saline by means of a syringe connected to the inflation arm 31. Irrigating fluid supplied via the irrigation arm 32 flows along the minor lumen 11 and emerges out of the patient end of the catheter via the eye 16. Urine flows into the main lumen 10 via the eyes 16, 17, 19 and 20 and is drained away via the coupling 3.

Although there is some constriction of the machine end of the shaft 1 in the coupling 30, this does not significantly reduce flow through the main lumen 10 because the internally-projecting ribs 13 and 14 are removed or reduced over the constricted portion. By comparison, in previous catheters, the ribs extend to the very end of the catheter and are sealed by means of plugs inserted into the minor lumens. This makes the shaft relatively stiff in the region of the ribs so that, when the shaft is constricted in the coupling, deformation takes place in the regions between the ribs and forces the ribs close together. This results in an appreciable narrowing of the opening out of the proximal end of the shaft compared with the present arrangement.

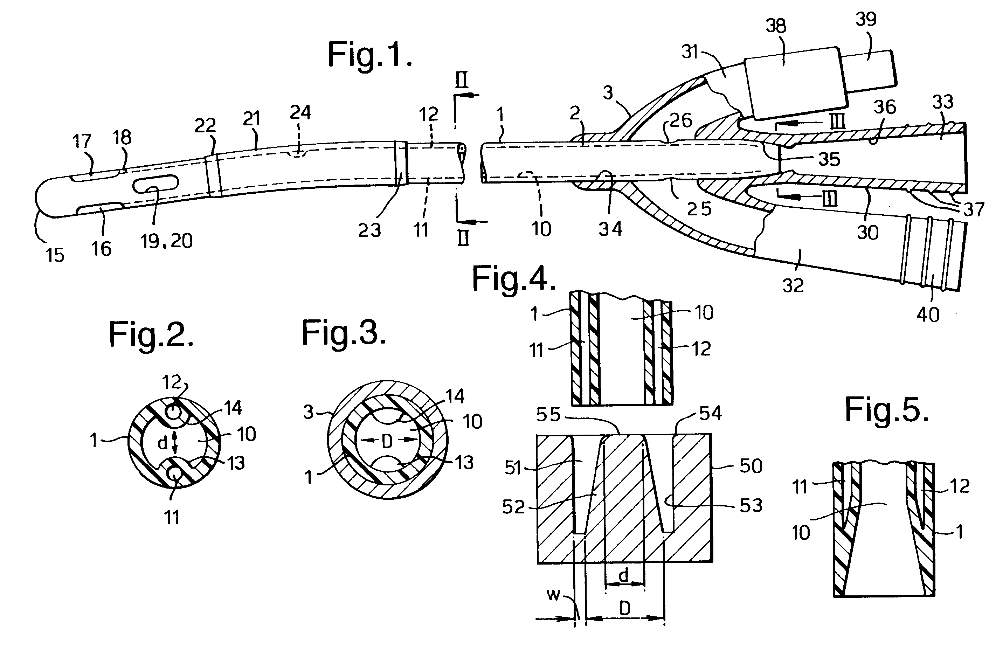

The method of forming the machine end of the shaft 1 will now be described with reference to Figures 4 and 5. The machine end 2 of the shaft 1 is initially provided with a square cut end so that the main lumen 10 and the minor lumens 11 and 12 are all open at the end; there is no need to fill or plug the minor lumens. The end of the shaft 1 is pushed into a heated die 50. The die 50 has a circular cavity 51 about 10mm deep and of annular shape with a central core 52. The outer wall 53 of the cavity 51 is cylindrical with a diameter equal to the external diameter of the shaft 1 and is bevelled at its upper end 54 to facilitate insertion of the shaft. The core 52 is of a generally conical shape with a top 55 having a diameter that is equal to the distance d between the two ribs 13 and 14 across a diameter of the shaft. The edge of the top 55 is bevelled or rounded to ease insertion of the core 52 into the main bore 10. At its lower end, the core 52 has a greater diameter D equal to the internal diameter of the shaft where it is not interrupted by the ribs 13 and 14. The width w of the annular cavity 51 at the lower end is, therefore equal to the wall thickness of the shaft.

The shaft 1 is pushed slowly into the die 50 so that its heat causes the material of the ribs 13 and 14 to soften and be deformed to the shape of the cavity. This results in the minor lumens 11 and 12 being occluded by the wall material of the shaft and produces a circular internal shape to the shaft at its tip with a smooth transition, along the depth of the cavity, as shown in Figure 5.

The openings 25 and 26 are cut into the minor lumens 11 and 12 either before or after the end-forming operation. The end-forming of the shaft can be carried out easily in production.

## Claims

1. A catheter having a shaft (1) with a main lumen (10), a minor lumen (11, 12) extending along a rib (13, 14) projecting internally into the main lumen and a coupling (3) into which the shaft is bonded at its machine end, the coupling making connection with the minor lumen via an opening (25, 26) in the wall of the shaft, characterised in that the rib (13, 14) at the machine end is heat formed outwardly into the wall of the shaft (1) so as to occlude the minor lumen (11, 12).

2. A catheter according to Claim 1, characterised in that the catheter has an inflatable cuff (21) encircling the shaft (1) close to the patient end, and that the minor lumen (12) opens into the cuff.

3. A catheter according to Claim 1 or 2, characterised in that the shaft (1) has two minor lumens (11 and 12) extending along respective ribs (13 and 14) projecting internally into the main lumen (10), that both ribs are heat formed outwardly at the machine end into the wall of the shaft so as to occlude both lumens (11 and 12), and that the coupling (3) makes connection with both minor lumens via respective openings (25 and 26) in the wall of the shaft.

4. A catheter according to Claim 3, characterised in that the coupling (3) has a main body (30) making connection with the main lumen (10) and two side arms (32 and 31) making connection with respective ones of the minor lumens (11 and 12).

5. A catheter according to any one of the preceding claims, characterised in that the patient end tip (15) of the catheter is closed, and that the catheter has an eye (16) cutting through the or a minor lumen (11) so that both the main lumen (10) and the minor lumen (11) open through the eye.

6. A catheter according to Claim 5 dependent on Claim 3 or 4, characterised in that the two minor lumens (11 and 12) are diametrically opposite one another, that the catheter has two eyes (16 and 17) diametrically opposite one another and cutting through both minor lumens, and that one of the minor lumens (12) is closed on the machine side of its eye (17).

7. A catheter according to Claim 6, characterised in that the catheter has a further two eyes (19 and 20), and that the further two eyes are displaced by 90° relative to the first two eyes (16 and 17) so that the further two eyes (19 and 20) do not cut the minor lumens (11 and 12).

8. A method of manufacture of a catheter including the steps of providing a shaft (1) with a main lumen (10) and a minor lumen (11, 12) extending along a rib (13, 14) projecting internally into the main lumen (10), occluding the minor lumen (11, 12), forming an opening (25, 26) into the minor lumen (11, 12) through the wall of the catheter at a point distally of the occlusion, and bonding the machine end of the shaft into a coupling (3), characterised in that the method includes the steps of heat forming the interior of the shaft (1) at the machine end to mould the rib (13, 14) outwardly into the wall of the shaft and occlude the minor lumen (11, 12).

9. A method according to Claim 8, characterised in that the shaft (1) is heat formed by pushing the machine end of the shaft into a heated die (50) having a central core (52).

## Patentansprüche

1. Ein Katheter mit einem Schaft (1) mit einem Hauptkanal (10), einem Nebenkanal (11, 12), der sich entlang einer Rippe (13, 14), welche nach innen in den Hauptkanal hineinragt, erstreckt, und einer Kupplung (3), in welche der Schaft an seinem maschinenseitigen Ende eingebunden ist, wobei die Kupplung eine Verbindung mit dem Nebenkanal über eine Öffnung (25, 26) in der Wand des Schaftes herstellt, **dadurch gekennzeichnet,** daß die Rippe (13, 14) am maschinenseitigen Ende durch Wärmebehandlung nach außen in die Wand des Schaftes (1) umgeformt ist, so daß der Nebenkanal (11, 12) eingeschlossen wird.

2. Katheter nach Anspruch 1, **dadurch gekennzeichnet,** daß der Kather eine aufblasbare Manschette (21) besitzt, welche den Schaft (1) nahe dem patientenseitigen Ende umgibt, und daß der Nebenkanal (12) in die Manschette öffnet.

3. Katheter nach Anspruch 1 oder 2, **dadurch gekennzeichnet,** daß der Schaft (1) zwei Nebenkanäle (11 und 12) besitzt, welche sich entlang jeweiliger Rippen (13 und 14), welche nach innen in den Hauptkanal (10) hineinragen, erstrecken, daß beide Rippen am maschinenseitigen Ende nach außen in die Wand des Schaftes wärmeverformt sind, damit beide Kanäle (11 und 12) eingeschlossen sind, und daß die Kupplung (3) eine Verbindung mit beiden Nebenkanälen über jeweilige Öffnungen (25 und 26) in der Wand des Schaftes herstellt.

4. Katheter nach Anspruch 3, **dadurch gekennzeichnet,** daß die Kupplung (3) einen Hauptrumpf (30) besitzt, der eine Verbindung mit dem Hauptkanal (10) herstellt und zwei Seitenarme (31 und 32) eine Verbindung mit jeweils einem der Nebenkanäle (11 und 12) herstellen.

5. Katheter nach einem der vorigen Ansprüche, **dadurch gekennzeichnet,** daß die patientenseitige Spitze 15 des Katheters geschlossen ist und daß der Katheter eine Öffnung (16) besitzt, welche durch den oder einen Nebenkanal (11) durchtritt, so daß sowohl der Hauptkanal (10) als auch der Nebenkanal (11) durch diese Öffnung öffnen.

6. Katheter nach Anspruch 5 in Abhängigkeit von Anspruch 3 oder 4, **dadurch gekennzeichnet,** daß die beiden Nebenkanale (11 und 12) einander diametral gegenüberliegen, daß der Katheter zwei Öffnungen (16 und 17) einander diametral gegenüber besitzt, die durch beide Nebenkanäle durchtreten, und daß einer der Nebenkanäle (12) am maschinenseitigen Ende seiner Öffnung (17) geschlossen ist.

7. Katheter nach Anspruch 6, **dadurch gekennzeichnet,** daß der Katheter zwei weitere Öffnungen (19 und 20) besitzt, und daß die beiden weiteren Öffnungen relativ zu den ersten beiden Öffnungen (16 und 17) um 90° versetzt sind, so daß die beiden weiteren Öffnungen (19 und 20) nicht die Nebenkanäle (11 und 12) schneiden.

8. Methode zur Herstellung eines Katheters unter Einschließung der Schritte zur Bereitstellung eines Schaftes (1) mit einem Hauptkanal (10) und einem Nebenkanal (11, 12), welcher entlang einer Rippe (13, 14) verläuft, die unter Einschließung des Nebenkanals (11, 12) nach innen in den Hauptkanal (10) hineinragt, zum Formen einer Öffnung (25, 26) in den Nebenkanal (11, 12) durch die Wand des Katheters an einem von der Einschließung entfernten Punkt, und zur Einbindung des maschinenseitigen Endes des Schaftes in eine Kupplung (3), **dadurch gekennzeichnet,** daß die Methode die Schritte einer Warmverformung des Inneren des Schaftes (1) am maschinenseitigen Ende zur Umformung der Rippe (13, 14) nach außen in die Schaftswand und zum Einschließen des Nebenkanals (11, 12) umfaßt.

9. Methode nach Anspruch 8, **dadurch gekennzeichnet,** daß der Schaft (1) durch Schieben des maschinenseitigen Endes des Schaftes in eine erhitzte Form (50) mit einem zentralen Kern (52) wärmeverformt wird.

## Revendications

1. Cathéter comportant une tige (1) avec une lumière principale (10), une lumière secondaire (11, 12) s'étendant le long d'une nervure (13, 14) saillante à l'intérieur de la lumière principale et un accouplement (3) dans lequel la tige est liée à son extrémité côté machine, l'accouplement établissant une connexion avec a lumière secondaire via une ouverture (25, 26) dans la paroi de la tige, caractérisé en ce que la nervure (13, 14) à l'extrémité côté machine est façonnée à chaud vers l'extérieur à l'intérieur de la paroi de la tige (1) afin d'obstruer la lumière secondaire (11, 12).

2. Cathéter selon la revendication 1, caractérisé en ce que le cathéter comporte une manchette gonflable (21) encerclant la tige (1), proche de l'extrémité côté patient, et en ce que la lumière secondaire (12) aboutit à l'intérieur de la manchette.

3. Cathéter selon la revendication 1 ou 2, caractérisé en ce que la tige (1) comporte deux lumières secondaires (11 et 12) s'étendant le long des nervures respectives (13 et 14) saillantes à l'intérieur de la lumière principale (10), en ce que les deux nervures sont façonnées à chaud vers l'extérieur à l'extrémité côté machine dans la paroi de la tige afin d'obstruer les deux lumières (11 et 12), et en ce que l'accouplement (3) établit une connexion avec les deux lumières secondaires via des ouvertures respectives (25 et 26) dans la paroi de la tige.

4. Cathéter selon la revendication 3, caractérisé en ce que l'accouplement (3) comporte un corps principal (30) établissant une connexion avec la lumière principale (10) et deux bras latéraux (32 et 31) établissant chacun une connexion avec une des lumières secondaires respectives (11 et 12).

5. Cathéter selon l'une quelconque des revendications précédentes, caractérisé en ce que le bout (15) de l'extrémité côté patient du cathéter est fermé, et en ce que le cathéter comporte un oeil (16) passant à travers la ou une lumière secondaire (11) pour que, à la fois, la lumière secondaire (11) et la lumière principale (10) s'ouvrent a travers l'oeil.

6. Cathéter selon la revendication 5 dépendant de la revendication 3 ou 4, caractérisé en ce que les deux lumières secondaires (11 et 12) sont diamétralement opposées l'une de l'autre, en ce que le cathéter comporte deux yeux (16 et 17) diamétralement opposés l'un de l'autre et passant à travers les deux lumières secondaires, et en ce qu'une des lumières secondaires (12) est fermée de son oeil (17) sur le côté machine.

7. Cathéter selon la revendication 6, caractérisé en ce que le cathéter comporte deux yeux supplémentaires (19 et 20), et en ce que lues deux yeux supplémentaires sont décalés de 90° par rapport aux deux premiers yeux (16 et 17) pour que les deux yeux supplémentaires (19 et 20) ne coupent pas les lumières secondaires (11 et 12).

8. Méthode de fabrication d'un cathéter incluant les étapes de réalisation d'une tige (1) avec une lumière principale (10) et une lumière secondaire (11, 12) s'étendant le long d'une nervure (13, 14) saillante à l'intérieur de la lumière principal (10), fermant la lumière secondaire (11, 12), formant une ouverture (25, 26) à l'intérieur de la lumière secondaire (11, 12) à travers la paroi du cathéter à un point distal de l'occlusion, et liant l'extrémité côté machine de la tige à l'intérieur d'un accouplement (3), caractérisée en ce que la méthode comporte les étapes de façonnage à chaud de l'intérieur de la tige (1) à l'extrémité côté machine pour mouler la nervure (13, 14) vers l'extérieur à l'intérieur de la paroi de la tige et obstruer la lumière secondaire (11, 12).

9. Méthode selon la revendication 8, caractérisée en ce que la tige (1) est façonnée à chaud en poussant l'extrémité côté machine de la tige à l'intérieur d'une matrice chauffée (50) ayant un noyau central (52).
